(19) Europäisches Patentamt
European Patent Office
Office européen des brevets

(11) EP 4 548 917 A1

(12) EUROPEAN PATENT APPLICATION
published in accordance with Art. 153(4) EPC

(43) Date of publication:
**07.05.2025 Bulletin 2025/19**

(21) Application number: **23831617.8**

(22) Date of filing: **30.06.2023**

(51) International Patent Classification (IPC):
***A61K 31/197*** (2006.01) ***A61K 9/14*** (2006.01)
***A61K 9/16*** (2006.01) ***A61K 9/20*** (2006.01)
***A61K 9/48*** (2006.01) ***A61K 31/22*** (2006.01)
***A61K 47/02*** (2006.01) ***A61K 47/04*** (2006.01)
***A61K 47/06*** (2006.01) ***A61K 47/10*** (2017.01)
***A61K 47/12*** (2006.01) ***A61K 47/14*** (2017.01)
***A61P 1/08*** (2006.01) ***A61P 7/06*** (2006.01)
***A61P 43/00*** (2006.01)

(52) Cooperative Patent Classification (CPC):
**A61K 9/14; A61K 9/16; A61K 9/20; A61K 9/48; A61K 31/197; A61K 31/22; A61K 47/02; A61K 47/06; A61K 47/10; A61K 47/12; A61K 47/14; A61P 1/08; A61P 7/06; A61P 43/00**

(86) International application number:
**PCT/JP2023/024334**

(87) International publication number:
**WO 2024/005178 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.07.2022 JP 2022107018**

(71) Applicant: **Kiyan Pharma Co., Ltd.**
**Fukuroi-shi, Shizuoka 437-0061 (JP)**

(72) Inventors:
- **DEKI Takeshi**
  **Fukuroi-shi, Shizuoka 437-0061 (JP)**
- **ISHII Kuniaki**
  **Fukuroi-shi, Shizuoka 437-0061 (JP)**
- **HORAGUCHI Haruhiko**
  **Fukuroi-shi, Shizuoka 437-0061 (JP)**
- **YAMASHITA Tomonori**
  **Fukuroi-shi, Shizuoka 437-0061 (JP)**

(74) Representative: **Kraus & Lederer PartGmbB**
**Thomas-Wimmer-Ring 15**
**80539 München (DE)**

# (54) NON-AQUEOUS PREPARATION WITH EXCELLENT STABILITY

(57) The present invention provides a non-aqueous preparation that comprises 5-ALA, a derivative thereof, or a salt of these, and that has excellent stability of the 5-ALA, the derivative, or the salt of these. A non-aqueous preparation according to the present invention comprises: a compound represented by the following general formula (I) or a salt thereof; a sugar or a sugar alcohol; and at least one substance selected from the group consisting of fatty acids, fatty acid esters, aliphatic hydrocarbons, and aliphatic alcohols with a melting point of 30-100°C. [In general formula (I), $R^1$ represents a hydrogen atom or an acyl group, and $R^2$ represents a hydrogen atom, a linear or branched alkyl group, a cycloalkyl group, an aryl group, or an aralkyl group.]

EP 4 548 917 A1

[Chemical Formula 1]

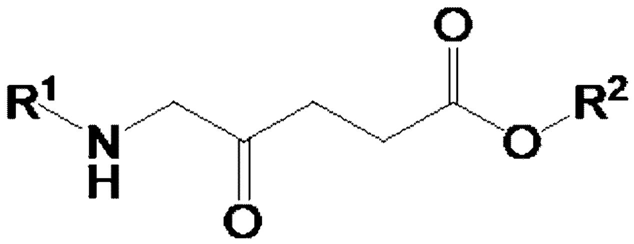

(I)

## Description

Technical Field

The present invention relates to a non-aqueous formulation having excellent stability.

Background Art

Aminolevulinic acid (5-ALA), derivatives thereof, and salts thereof are used as agents for photodynamic diagnosis for visualizing muscle layer non-invasive bladder cancer at the time of transurethral bladder tumor resection, health foods, and the like. In addition, Patent Documents 1 to 3 also disclose a preventive and/or therapeutic agent containing ALAs for side effects of anticancer agents (Patent Document 1), a preventive and/or therapeutic agent containing ALAs for a hangover (Patent Document 2), an ameliorating or preventive agent containing ALA for cancerous anemia (Patent Document 3), and the like.

In general, a compound useful as an active ingredient in a medicine, a nutritional ingredient in a health food, or the like is formulated together with an additive such as an excipient, a binder, a disintegrant, or a lubricant, and is taken as a tablet or a granule that is easy to administer orally. In such formulations, it is extremely important to secure the storage stability of the active ingredients and the nutritional ingredients in the formulation from the viewpoint of exerting expected drug efficacy, avoiding unexpected side effects, preventing deterioration in commercial properties due to discoloration, and the like.

However, the formulation containing 5-ALA has a problem that degradation of 5-ALA over time is induced depending on the form of the formulation and the type of additives. For example, Patent Document 4 describes that when a formulation containing ALAs has been developed, they have faced problems that in a case where ALAs and an iron compound coexist in a formulation containing moisture, the stability of the ALAs in the formulation is deteriorated, and the concentration of ALA is reduced in a short period of time. Thus, it is difficult to develop a formulation containing 5-ALA, and a non-aqueous formulation having excellent stability of 5-ALA has not yet been known.

Citation List

Patent Document

Patent Document 1: WO 2013/054756
Patent Document 2: WO 2013/084816
Patent Document 3: WO 2013/054770
Patent Document 4: WO 2018/043240
Patent Document 5: JP 2014-189492 A

Non-Patent Document

Non-Patent Document 1: Bunke, A., et al., Degradation Mechanism and Stability of 5-Aminolevulinic Acid, JOURNAL OF PHARMACEUTICAL SCIENCES, VOL. 89, NO. 10, OCTOBER 2000, pp. 1335-1341

Summary of the Invention

Problems to be Solved by the Invention

An object of the present invention is to provide a non-aqueous formulation containing 5-ALA or a derivative thereof or a salt thereof and having excellent stability of 5-ALA or the derivative thereof or the salt thereof.

Means for Solving the Problems

In some aspects, the present invention provides the following [1] to [9].

[1] A non-aqueous formulation containing:

a compound of the following formula (I), or a salt thereof:

[Chemical Formula 1]

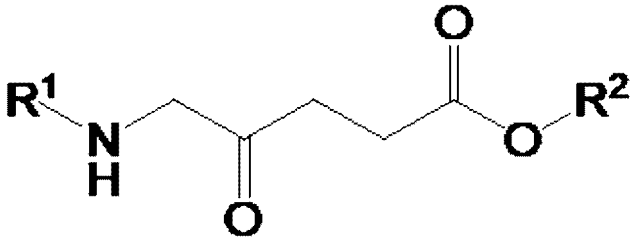

(I)

in which $R^1$ is a hydrogen atom or an acyl group, and $R^2$ is a hydrogen atom, a linear or branched alkyl group, a cycloalkyl group, an aryl group, or an aralkyl group;
a sugar or sugar alcohol; and
at least one component having a melting point of 30 to 100°C selected from the group consisting of fatty acids, fatty acid esters, aliphatic hydrocarbons, and aliphatic alcohols.

[2] The non-aqueous formulation according to [1], in which the sugar or sugar alcohol is one or more selected from the group consisting of D-mannitol, erythritol, xylitol, sorbitol, lactose, trehalose, isomalt hydrate, and maltitol.
[3] The non-aqueous formulation according to [1] or [2], in which the fatty acid ester is a fatty acid ester of a polyhydric alcohol.
[4] The non-aqueous formulation according to [1] to [3], in which the fatty acid ester is one or more selected from the group consisting of sucrose fatty acid esters and glycerin fatty acid esters.
[5] The non-aqueous formulation according to any one of [1] to [4], in which the fatty acid is a saturated fatty acid having 10 to 24 carbon atoms.
[6] The non-aqueous formulation according to any one of [1] to [5], in which the content of the compound of the formula (I) or the salt thereof is 5 to 80 mass% based on the total amount of the non-aqueous formulation.
[7] A product including the non-aqueous formulation of any one of [1] to [6] and a package that accommodates the non-aqueous formulation in a dry state.
[8] The product according [7], in which the non-aqueous formulation has the form of tablets, capsules, powder, granules, or fine granules.
[9] The product according to [7] or [8], in which the non-aqueous formulation is brought into a dry state by one or more desiccants selected from the group consisting of silica gel, silica alumina gel (allophane and the like), natural zeolite, synthetic zeolite, calcium chloride, quicklime (calcium oxide), bentonite clay (montmorillonite and the like), magnesium chloride, magnesium oxide, calcium sulfate, activated alumina, alumina, bauxite, anhydrous calcium sulfate and water-absorbent clay, and mixtures of these components and activated carbon.

Effects of the Invention

According to the present invention, it is possible to provide a non-aqueous formulation containing 5-ALA or a derivative thereof or a salt thereof and having excellent stability of 5-ALA or the derivative thereof or the salt thereof.

Description of Embodiments

Hereinafter, some embodiments of the present invention will be described in detail. However, the present invention is not limited to the following embodiments.
The non-aqueous formulation according to an embodiment contains a compound of the formula (I) or a salt thereof:

[Chemical Formula 2]

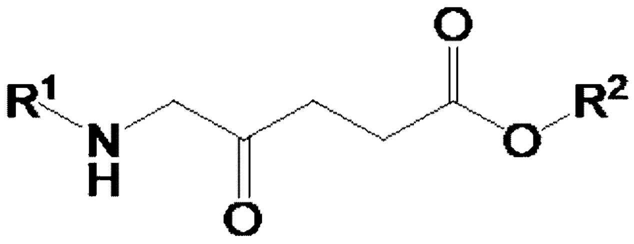

(I)

In the formula, $R^1$ is a hydrogen atom or an acyl group, and $R^2$ is a hydrogen atom, a linear or branched alkyl group, a cycloalkyl group, an aryl group, or an aralkyl group.

Hereinafter, the compound of the formula (I) or the salt thereof may be collectively referred to as "5-ALA compound". The compounds represented by the general formula (I) are composed of 5-ALA (5-aminolevulinic acid) and 5-ALA derivatives (compounds represented by the general formula (I) other than 5-ALA). Therefore, the 5-ALA compound can also be referred to as 5-ALA, 5-ALA derivative, or salts thereof.

First, 5-ALA (5-aminolevulinic acid) will be described. 5-ALA is a compound in which $R^1$ and $R^2$ are both hydrogen atoms in the formula (I), and is a type of amino acid.

Next, the 5-ALA derivative will be described. In the 5-ALA derivative, $R^1$ may be a hydrogen atom, and $R^2$ may be a linear or branched alkyl group, a cycloalkyl group, an aryl group, or an aralkyl group, or $R^1$ may be an acyl group, and $R^2$ may be a hydrogen atom, a linear or branched alkyl group, a cycloalkyl group, an aryl group, or an aralkyl group.

Examples of the acyl group in $R^1$ include linear or branched alkanoyl groups having 1 to 8 carbon atoms, such as formyl, acetyl, propionyl, butyryl, isobutyryl, valeryl, isovaleryl, pivaloyl, hexanoyl, octanoyl, and benzylcarbonyl groups, and aroyl groups having 7 to 14 carbon atoms, such as benzoyl, 1-naphthoyl, and 2-naphthoyl groups.

Examples of the alkyl group in $R^2$ include linear or branched alkyl groups having 1 to 8 carbon atoms, such as methyl, ethyl, propyl, isopropyl, butyl, isobutyl, sec-butyl, tert-butyl, pentyl, isopentyl, neopentyl, hexyl, heptyl, and octyl groups.

Examples of the cycloalkyl group in $R^2$ include cycloalkyl groups having 3 to 8 carbon atoms in which a saturated or partially unsaturated bond may be present, such as a cyclopropyl, cyclobutyl, cyclopentyl, cyclohexyl, cycloheptyl, cyclooctyl, cyclododecyl, and 1-cyclohexenyl groups.

Examples of the aryl group in $R^2$ include aryl groups having 6 to 14 carbon atoms, such as phenyl, naphthyl, anthryl, and phenanthryl groups.

The aralkyl group in $R^2$ can have an aryl moiety exemplified in the same manner as the above-described aryl group, and can have an alkyl moiety exemplified in the same manner as the above-described alkyl group. Specific examples thereof include aralkyl groups having 7 to 15 carbon atoms, such as benzyl, phenethyl, phenylpropyl, phenylbutyl, benzhydryl, trityl, naphthylmethyl, and naphthylethyl groups.

The 5-ALA derivative is preferably a compound in which $R^1$ is a formyl, acetyl, propionyl, or butyryl group, or the like. In addition, the 5-ALA derivative is preferably a compound in which $R^2$ is a methyl, ethyl, propyl, butyl, or pentyl group, or the like. Preferable examples of the 5-ALA derivative include compounds in which the combination of $R^1$ and $R^2$ is a combination of formyl and methyl, acetyl and methyl, propionyl and methyl, butyryl and methyl, formyl and ethyl, acetyl and ethyl, propionyl and ethyl, or butyryl and ethyl.

The 5-ALA derivative may be a prodrug of 5-ALA. The prodrug of 5-ALA is a compound that produces 5-ALA by being metabolized in vivo or the like. Examples of the prodrug of 5-aminolevulinic acid include esters of 5-aminolevulinic acid, such as 5-aminolevulinic acid methyl ester, 5-aminolevulinic acid ethyl ester, 5-aminolevulinic acid propyl ester, 5-aminolevulinic acid butyl ester, and 5-aminolevulinic acid pentyl ester, in which $R^1$ is a hydrogen atom and $R^2$ is a linear or branched C1 to C5 alkyl group.

Next, salts of 5-ALA or 5-ALA derivatives will be described. The salt of 5-ALA or the 5-ALA derivative may be a pharmacologically acceptable salt. Examples of the salt include acid addition salts, metal salts, ammonium salts, and organic amine addition salts and the like. Examples of the acid addition salts include inorganic acid salts such as hydrochlorides, hydrobromides, hydroiodides, phosphates, nitrates, and sulfates; and organic acid addition salts such as formates, acetates, propionates, toluenesulfonates, succinates, oxalates, lactates, tartrates, glycolates, methanesulfonates, butyrates, valerates, citrates, fumarates, maleates, and malates. Examples of the metal salts include alkali metal salts such as lithium salts, sodium salts, and potassium salts, alkaline earth metal salts such as magnesium salts and calcium salts, aluminum salts, and zinc salts. Examples of the ammonium salts include alkylammonium salts such as ammonium salts and tetramethylammonium salts. Examples of the organic amine addition salts include triethylamine salts, piperidine salts, morpholine salts, and toluidine salts.

The 5-ALA compound is preferably one or more selected from the group consisting of 5-ALA and 5-ALA esters (5-ALA methyl ester, 5-ALA ethyl ester, 5-ALA propyl ester, 5-ALA butyl ester, 5-ALA pentyl ester, and the like), and hydrochlorides, phosphates, and sulfates thereof. The 5-ALA compound is more preferably 5-ALA hydrochloride and/or 5-ALA phosphate.

The content of the 5-ALA compound may be 5 mass% or more, 20 mass% or more, or 40 mass% or more, and may be 80 mass% or less, 70 mass% or less, or 65 mass% or less, based on the total amount of the non-aqueous formulation. The content of the 5-ALA compound is preferably 5 to 80 mass%, more preferably 20 to 70 mass%, and particularly preferably 40 to 65 mass%, based on the total amount of the non-aqueous formulation.

The non-aqueous formulation according to one embodiment can contain a sugar or sugar alcohol in addition to the 5-ALA compound described above. The sugar or sugar alcohol is one or more selected from the group consisting of monosaccharides, disaccharides, oligosaccharides (sugars having a molecular weight of 300 to 3000, excluding those corresponding to disaccharides.), and sugar alcohols thereof.

Examples of the monosaccharides include glucose and fructose, and examples of the sugar alcohols of monosaccharides (also referred to as "monosaccharide alcohols".) include D-mannitol, erythritol, xylitol, and sorbitol.

Examples of the disaccharides include lactose (for example, lactose hydrate, anhydrous lactose, spray-dried lactose, fluidized bed granulated lactose, and isomerized lactose), sucrose, trehalose, and maltose, and examples of the sugar alcohols of disaccharides (also referred to as "disaccharide alcohols".) include isomalt hydrate, lactitol (reduced lactose), and maltitol. The disaccharide may be contained as refined white sugar, white sugar, or the like. In addition, the refined white sugar and white sugar include sucrose. Also, the disaccharide alcohol may be contained as reduced maltose syrup or the like. The reduced maltose syrup includes monosaccharide alcohols, disaccharide alcohols (maltitol), and sugar alcohols of oligosaccharides (also referred to as "oligosaccharide alcohols".). When the non-aqueous formulation contains reduced maltose syrup, it can be said that a monosaccharide alcohol and an oligosaccharide alcohol are contained as the reduced maltose syrup in addition to the disaccharide alcohol.

The oligosaccharide may also be referred to as oligosaccharide. Examples of the oligosaccharide include fructo-oligosaccharides. The oligosaccharide may be a sugar in which three or more and ten or less monosaccharides are bonded.

The non-aqueous formulation may contain only one or two or more of these sugars or sugar alcohols. The sugar or sugar alcohol is preferably at least one selected from the group consisting of monosaccharides, disaccharides, oligosaccharides, and sugar alcohols thereof, and more preferably at least one selected from the group consisting of sugar alcohols of monosaccharides, sugar alcohols of disaccharides, and sugar alcohols of oligosaccharides. Also, the sugar or sugar alcohol may be one or more selected from the group consisting of D-mannitol, erythritol, xylitol, sorbitol, lactose, trehalose, isomalt hydrate, and maltitol.

The content of the sugar or sugar alcohol may be 10 mass% or more, 20 mass% or more, 30 mass% or more, or 33 mass% or more, and may be 95 mass% or less, 80 mass% or less, 70 mass% or less, or 60 mass% or less, based on the total amount of the non-aqueous formulation. The content of the sugar or sugar alcohol may be 10 to 95 mass%, and is preferably 20 to 80 mass%, more preferably 30 to 70 mass%, and still more preferably 33 to 60 mass%, based on the total amount of the non-aqueous formulation.

In addition to the 5-ALA compound described above, the non-aqueous formulation according to an embodiment can contain at least one component having a melting point of 30 to 100°C selected from the group consisting of fatty acids, fatty acid esters, aliphatic hydrocarbons, and aliphatic alcohols (hereinafter, also referred to as "low melting point compound".). Here, the melting point can be measured by the method described in "Japanese Pharmacopoeia Eighteenth Edition, General Tests, 2. Physical Methods, 2. 60 Melting Point Determination". Each of the low melting point compounds may be water-insoluble or poorly watersoluble.

The fatty acid as the low melting point compound is a fatty acid having a melting point of 30 to 100°C. The melting point of the fatty acid may be 40°C or higher, or 50°C or higher, and may be 90°C or lower, or 80°C or lower. The fatty acid is preferably a saturated fatty acid having 10 to 24 carbon atoms, and the number of carbon atoms of the saturated fatty acid may be 12 to 24 or 14 to 18. Examples of the saturated fatty acid having 10 to 24 carbon atoms include decanoic acid (capric acid), dodecanoic acid (lauric acid), tetradecanoic acid (myristic acid), hexadecanoic acid (palmitic acid), heptadecanoic acid (margaric acid), octadecanoic acid (stearic acid), eicosanoic acid (arachidic acid), docosanoic acid (behenic acid), and tetracosanoic acid (lignoceric acid). The fatty acid is particularly preferably stearic acid. The fatty acid may be contained as a wood wax.

The fatty acid ester as the low melting point compound is a fatty acid ester having a melting point of 30 to 100°C. The melting point of the fatty acid ester may be 40°C or higher or 50°C or higher, and may be 90°C or lower or 80°C or lower. The fatty acid ester may be, for example, a fatty acid ester of a polyhydric alcohol. Examples of the fatty acid ester of a polyhydric alcohol include sucrose fatty acid esters, glycerin fatty acid esters, propylene glycol fatty acid esters, and sorbitan fatty acid esters. The fatty acid ester of a polyhydric alcohol is particularly preferably a sucrose fatty acid ester or a glycerin fatty acid ester or a mixture thereof.

The sucrose fatty acid ester is an ester of sucrose, which is a hydrophilic group, and fatty acids derived from vegetable fats and oils, which are lipophilic groups, and is also called a sugar ester. Sucrose is also called sucrose, is a sugar formed of two monosaccharides, glucose and fructose, and has eight hydroxyl groups. The sucrose fatty acid ester is one in which one or more hydroxyl groups among hydroxyl groups possessed by sucrose are substituted with fatty acid.

The sucrose fatty acid ester may be a monoester in which one of hydroxyl groups of sucrose is substituted with a fatty acid, an ester in which two or more and eight or less of hydroxyl groups of sucrose are substituted with fatty acids (diester, triester, octaester, or the like), or a mixture thereof.

The fatty acid constituting the sucrose fatty acid ester may be saturated fatty acid or unsaturated fatty acid. In addition, the fatty acid constituting the sucrose fatty acid ester may be linear or branched. The fatty acid constituting the sucrose fatty acid ester may be fatty acids having 10 to 24 carbon atoms, and is preferably fatty acids having 12 to 22 carbon atoms or 18 to 22 carbon atoms. Examples of the fatty acid having 18 to 22 carbon atoms include octadecanoic acid (stearic acid), eicosanoic acid (arachidic acid), docosanoic acid (behenic acid), and erucic acid.

The glycerin fatty acid ester is an ester of glycerin and fatty acids. Glycerin is a trivalent sugar alcohol and has three hydroxyl groups. The glycerin fatty acid ester is one in which one or more hydroxyl groups among hydroxyl groups possessed by glycerin are substituted with fatty acid. The glycerin fatty acid ester is preferably a monoester in which one of hydroxyl groups of glycerin is substituted with a fatty acid. The glycerin fatty acid ester may be contained as a fat and oil. Examples of the fat and oil include vegetable fats such as cacao fat and hydrogenated vegetable oils such as hydrogenated rapeseed oil and hydrogenated castor oil. Hydrogenated vegetable oils are oils and fats solidified at room temperature, the ratio of saturated fatty acids having a higher melting points is increased by hydrogenation of vegetable oils and fats that are liquid at room temperature because they contain a large amount of unsaturated fatty acids having a relatively low melting points.

The aliphatic hydrocarbon as the low melting point compound is an aliphatic hydrocarbon having a melting point of 30 to 100°C. The melting point of the aliphatic hydrocarbon may be 40°C or higher or 50°C or higher, and may be 90°C or lower or 80°C or lower. The aliphatic hydrocarbon may be a saturated aliphatic hydrocarbon. The aliphatic hydrocarbon may be contained as a mineral oil. The mineral oil is a hydrocarbon refined from petroleum and minerals. Examples of the mineral oil include paraffin (petroleum), microcrystalline wax (petroleum), and ceresin (ground wax).

The aliphatic alcohol as the low melting point compound is an aliphatic alcohol having a melting point of 30 to 100°C. The melting point of the aliphatic alcohol may be 40°C or higher or 50°C or higher, and may be 90°C or lower or 80°C or lower. The aliphatic alcohol may be a saturated aliphatic alcohol or an unsaturated aliphatic alcohol. Also, the aliphatic alcohol may be linear or branched. The number of carbon atoms of the aliphatic alcohol may be 14 to 24 or 15 to 20. Examples of the aliphatic alcohol include cetanol and stearyl alcohol.

The low melting point compound may be contained as vegetable or animal wax. Examples of the vegetable wax include carnauba wax (carnauba palm). Examples of the animal wax include beeswax.

The low melting point compound may be at least one selected from the group consisting of fatty acid esters of polyhydric alcohols and fatty acids having a melting point of 30 to 100°C.

The low melting point compound may be 0.1 mass% or more, 0.5 mass% or more, or 1 mass% or more, and may be 20 mass% or less, 10 mass% or less, or 5 mass% or less, based on the total amount of the non-aqueous formulation. The content of the low melting point compound may be 0.1 to 20 mass%, preferably 0.5 to 10 mass%, and more preferably 1 to 5 mass%, based on the total amount of the non-aqueous formulation.

The non-aqueous formulation may contain a binder. Examples of the binder include crystalline cellulose, hypromellose, hydroxyalkyl cellulose such as hydroxypropyl cellulose and hydroxyethyl cellulose, and gum arabic. The non-aqueous formulation may contain one or two or more of these binders. The content of the binder may be 0.1 mass% or more, 0.5 mass% or more, or 1 mass% or more, and may be 20 mass% or less, 10 mass% or less, or 5 mass% or less, based on the total amount of the non-aqueous formulation. The content of the binder may be 0.1 to 20 mass%, preferably 0.5 to 10 mass%, and more preferably 1 to 5 mass%.

The non-aqueous formulation may further contain other additives commonly used in the field of formulation as long as the effects of the present invention are not affected. Examples of the other additives include excipients, disintegrants, lubricants, buffering agents, fluidizing agents, antioxidants, stabilizers, surfactants, sweetening agents, flavoring agents, fragrances, colorants, and film coating agents. The non-aqueous formulation may contain one or more of these additives.

Examples of the excipient include magnesium aluminate metasilicate, anhydrous calcium phosphate, anhydrous calcium hydrogen phosphate, calcium silicate, calcium lactate, precipitated calcium carbonate, calcium silicate, and calcium lactate.

Examples of the disintegrant include carmellose, carmellose calcium, carmellose sodium, low-substituted hydroxypropyl cellulose, crospovidone, croscarmellose sodium, sodium carboxymethyl starch, and sodium starch glycolate.

Examples of the lubricant include stearates such as magnesium stearate and calcium stearate. The melting point of stearate is generally 100°C or higher.

Examples of the buffer include organic acids such as carboxylic acids (succinic acid, fumaric acid, citric acid, tartaric acid, and the like), amino acids (glutamic acid, glutamine, glycine, aspartic acid, alanine, arginine, and the like), and ascorbic acid (excluding fatty acids having a melting point of 30 to 100°C.) and salts thereof; inorganic acids such as phosphoric acid and boric acid and salts thereof; metal hydroxides such as magnesium hydroxide; and metal oxides such

as magnesium oxide and zinc oxide.

Examples of the fluidizing agent include anhydrous calcium silicate, hydrous silicon dioxide, stearic acid, magnesium stearate, calcium stearate, and talc.

Examples of the antioxidant include citric acid, sodium nitrite, ascorbic acid, L-ascorbic acid stearic acid ester, sodium hydrogen nitrite, sodium nitrite, alpha thioglycerol, edetate sodium, erythorbic acid, cysteine hydrochloride, dry sodium sulfite, potassium dichloroisocyanurate, soybean lecithin, sodium thioglycolate, sodium thiomalate, natural vitamin E, tocopherol, d-δ-tocopherol, tocopherol acetate, concentrated mixed tocopherol, ascorbic acid palmitate, sodium pyrosulfite, butyl hydroxyanisole, 1,3-butylene glycol, benzotriazole, pentaerythrityl-tetrakis[3-(3,5-dit-butyl-4-hydroxyphenyl)propionate], 2-mercaptobenzimidazole, propyl gallate, and dibutylhydroxytoluene.

Examples of the stabilizer include citric acid and salts thereof (citric acid, citric acid hydrate, calcium citrate, sodium citrate hydrate, sodium dihydrogen citrate, disodium citrate, and the like).

Examples of the surfactant include polysorbate 80, sodium lauryl sulfate, and polyoxyethylene-hydrogenated castor oil.

Examples of the sweetening agent include saccharin sodium, glycyrrhizic acid, aspartame, stevia, and sormatin.

Examples of the flavoring agent include citric acid, tartaric acid, and malic acid, and the like.

Examples of the fragrance include lemon, lemon lime, orange, and menthol, and the like.

Examples of the colorant include iron sesquioxide, yellow iron sesquioxide, edible tar dyes and aluminum lakes thereof, riboflavin, and sodium copper chlorophyllin.

Examples of the film coating agent include hypromellose, polyvinyl alcohol, a methacrylic acid copolymer, aminoalkyl methacrylate copolymers, ammonioalkyl methacrylate copolymers, and ethyl cellulose.

The non-aqueous formulation may contain one other additive, or may contain two or more other additives in combination. An appropriate amount can be appropriately added. The content of other additives can be appropriately adjusted within a range not affecting the effect of the present invention, and may be, for example, 0.5 mass% or less based on the total amount of the non-aqueous formulation.

The non-aqueous formulation may contain a fixed amount of water. Specifically, the water content of the non-aqueous formulation is 20 mass% or less, preferably 7 mass% or less, more preferably 3 mass% or less, and still more preferably 1 mass% or less, based on the total amount of the non-aqueous formulation.

The non-aqueous formulation may, for example, have the form of fine granules, granules, tablets (for example, uncoated tablets, film-coated tablets, chewable tablets, or orally disintegrating tablets), capsules, powders, or dry syrups. The non-aqueous formulation is preferably a tablet. In addition, the non-aqueous formulation may be taken together with water at the time of taking. The non-aqueous formulation may be taken directly with water or may be taken after being added to water and made into a solution or suspension.

The non-aqueous formulation of the present invention may be a compounding agent further containing another active ingredient different from the 5-ALA compound. In this case, the dosage form of the non-aqueous formulation may be an ordinary tablet, a laminated tablet, a dry-coated tablet, a tablet or a granule containing coated granules, a coated tablet containing a principal agent in a film layer, or the like. The laminated tablet may be a bilayer tablet including a first layer and a second layer, and may be a multilayer tablet having one or more intermediate layers provided between the first layer and the second layer in addition to the first layer and the second layer (tri-layer tablet, four-layer tablet, five-layer tablet, or the like). The dry-coated tablet may be a dry-coated tablet having only an inner core and an outer layer, or may be a dry-coated tablet having an inner core, an outer layer, and an intermediate layer provided between the inner core and the outer layer. In these compounding agents, the 5-ALA compound, the sugar or sugar alcohol, and at least one component having a melting point of 30°C or higher selected from the group consisting of fatty acid esters of polyhydric alcohols and fatty acids may be contained only in a part of the layer, the inner core, or the outer shell.

The non-aqueous formulation according to an embodiment can be produced by a method including, for example, a pulverization process, a mixing process, a granulation process, a drying process, a molding (tableting) process, a film coating process, a crystallization process, and the like. The method for producing a non-aqueous formulation may be a method including some or all of these processes.

In the pulverization process, raw materials of the non-aqueous formulation are pulverized. The raw materials of the non-aqueous formulation contain the 5-ALA compound, the sugar or sugar alcohol, at least one low melting point compound, and at least one or more selected from the group consisting of other additives added as necessary. Device and means for pulverization are not particularly limited. Examples of the pulverizing device include a hammer mill, a ball mill, a jet mill, and a colloid mill. The pulverization conditions can be appropriately selected.

In the mixing process, the raw materials are mixed. The mixing process is not particularly limited in terms of device and means as long as the components can be uniformly mixed, and may be a process performed after the pulverization process or simultaneously with the pulverization process.

In the granulation process, the raw materials are granulated. Device and means for granulation are not particularly limited. The granulation process may be a wet granulation process using a solvent such as water or ethanol, or may be a dry granulation process. In the wet granulation process, it is preferable to use ethanol as the solvent, and it is

particularly preferable to use 90% or more of ethanol. Examples of the granulation method used in the wet granulation include a high-speed stirring granulation method, a crushing (pulverization) granulation method, a fluidized bed granulation method, an extrusion granulation method, a rolling granulation method, and a spray drying granulation method. In the granulation process, a high-speed stirring granulation method is preferably used.

In the drying process, for example, the granulated product obtained in the granulation process is dried. As device and means for drying, commonly used device and means can be used. In the drying process, it is preferable to perform drying under reduced pressure and/or in an environment of 60°C or lower. Examples of the drying device include a ventilation dryer, a vacuum dryer, a vacuum dryer, and a fluidized bed granulating dryer. The drying process may include a process of sieving (sizing) the granulated product using a sieve, a coal mill, or the like after drying.

In the molding (tableting) process, for example, the raw materials after the mixing process or the granulated product obtained in the granulation process is molded. In this case, the lubricant may be added to the granulated product before molding. Device and means for molding are not particularly limited. In the molding process, for example, compression molding is performed using a tableting device such as a rotary tableting machine or an oil press. The tableting conditions can be appropriately adjusted. The hardness of the tableted product obtained by the molding process may be any degree as long as it is not damaged in the production and distribution process, and may be, for example, 40 to 200 N.

In the film coating process, for example, the surface of the tableted product obtained in the molding process is subjected to film coating. Examples of the film coating method include pan coating and dip coating, and the like. In the film coating process, for example, one or a combination of two or more of the above-described film coating agents can be added to the tableted product. The coating rate (content of the film coating agent) of the non-aqueous formulation after the film coating process may be, for example, 1 mass% to 10 mass% based on the total amount of the non-aqueous formulation. In the film coating process, the tableted product during and after the film coating may be dried.

In the crystallization process, crystallization of the 5-ALA compound is promoted. In preparing a formulation, the ratio of crystals of the 5-ALA compound may decrease, and in such a case, it is preferable to perform the present crystallization process. Examples of the method for promoting crystallization include irradiation treatment with radio waves such as microwaves and low frequency, and thermal electron irradiation treatment. In the radio wave irradiation treatment, for example, radio waves of 10 MHz to 25 GHz may be emitted.

In this case, the treatment time is appropriately adjusted depending on the initial ratio of crystals, the type of additive, and the like, but may be, for example, 10 seconds to 60 minutes. In the radio wave irradiation treatment, radio waves of 10 kHz to 600 kHz may be emitted. In this case, the treatment time is appropriately adjusted depending on the initial ratio of crystals, the type of additive, and the like, but may be, for example, 10 seconds to 24 hours. The irradiation of the radio wave may be performed continuously or intermittently.

Since the non-aqueous formulation described above contains the 5-ALA compound, the sugar or sugar alcohol, and at least one low melting point compound, the 5-ALA compound is excellent in stability. The present inventors have found that the 5-ALA compound in the non-aqueous formulation is stabilized by containing the 5-ALA compound, the sugar or sugar alcohol and/or at least one low melting point compound in the non-aqueous formulation. Therefore, the non-aqueous formulation described above can also be said to be a stabilized non-aqueous formulation. An embodiment of the present invention is also a method for stabilizing a non-aqueous formulation, including a step of containing the 5-ALA compound, the sugar or sugar alcohol and/or at least one low melting point compound in the non-aqueous formulation.

The non-aqueous formulation may be contained in a package. Another embodiment of the present invention is a product including a non-aqueous formulation and a package containing the non-aqueous formulation in a dry state.

The package may be a package that can suppress substantial entry of moisture from the outside of the package in a state of normal handling, transportation, storage, or the like, and includes a "container", for example, a "tight container" and a "hermetic container" defined in Japanese Pharmacopoeia Eighteenth Edition, General Information, G0, Basic Concepts on Pharmaceutical Quality. The shape of the package may be a standard size container such as a jar or a bottle, or may be an indefinite shape such as strip packaging (SP) package, press through packaging (PTP) package, or bag-shaped package (for example, pillow type package and stick package). In the product, these packages may be used singly or in combination of two or more thereof. For example, in a product, the non-aqueous formulation may be contained in a primary package (for example, PTP package), and the primary package containing the non-aqueous formulation may be further contained in a secondary package (for example, pillow type package).

From the viewpoint of protecting the product from the effect of moisture, the material of the package is preferably a material that does not substantially permeate moisture or has a moisture-proof function. Here, the phrase "does not substantially permeate moisture" means that "does not permeate moisture in an amount that affects the non-aqueous formulation contained in the package". As the material of package, a material used for a package containing contents weak to moisture in the fields of pharmaceuticals and foods can be appropriately used.

Examples of the material of the jar or bottle include glass; resins such as polyester, polypropylene, polyethylene (including low density polyethylene (LDPE) and high density polyethylene (HDPE)), polycarbonate, and polystyrene; resins containing a desiccant in such resins (desiccant-containing resins); and metals such as aluminum. The jar may also

have a plug or lid. Examples of the material of the plug or lid include plastics such as polyester, polyethylene, polycarbonate, polystyrene, and polypropylene; and metals such as aluminum. A bottle made of plastic kneaded with a desiccant is also called a bottle with moisture absorption function.

Examples of the material of the SP package, the PTP package, and the bag-shaped packages (pillow type package, stick package, and the like) include resins such as biaxially stretched polypropylene (OPP), biaxially stretched polyester (PET), glucose-modified PET (PET-G), biaxially stretched nylon (ONy, PA), polyethylene (including low density polyethylene (LDPE) and linear low density polyethylene (L-LDPE)), ethylene-vinyl acetate copolymer (EVA), unstretched polypropylene (CPP, IPP), ionomer resin (IO), ethylene-methacrylic acid copolymer (EMAA), polyacrylonitrile (PAN), biaxially stretched polyvinylidene chloride (PVDC), ethylene-vinyl alcohol copolymer resin (EVOH), polyvinyl chloride (PVC), cyclic polyolefin (COC), unstretched nylon (CNy), polycarbonate (PC), polystyrene (PS), and hard vinyl chloride (VSC); resins containing a desiccant in such resins (desiccant-containing resins); metals such as aluminum (AL); cellophane; and paper.

The material of package can be used singly or in combination of two or more thereof. The package may be, for example, a laminate in which two or more layers made of different materials are laminated. Examples of the laminate having a two-layer structure include a laminate of the above-described metal, cellophane, or paper and the above-described resin or desiccant-containing resin. The laminate may have a moisture-proof layer that suppresses permeation of moisture as an outer layer or an intermediate layer, and may have an absorption layer having a drying function as an inner layer or an intermediate layer. Examples of the material constituting the absorption layer having a drying function include a desiccant-containing resin.

In the product according to one embodiment, the package contains the non-aqueous formulation in a dry state. Here, the phrase "contains ... in a dry state" refers to a state in which the non-aqueous formulation is maintained in a dry state by package, and includes a case where the package itself is made of a moisture-impermeable/water-impermeable material and a case where the non-aqueous formulation is packaged together with a desiccant. Examples of those in which the package itself is impermeable to moisture and impermeable to water, in addition to packages made of metal, include laminates of metal, cellophane, or paper and a desiccant-containing resin.

The desiccant may be a desiccant that can be usually used for formulation. The desiccant may be one or more selected from the group consisting of silica gel, silica alumina gel (allophane and the like), natural zeolite, synthetic zeolite, calcium chloride, quicklime (calcium oxide), bentonite clay (montmorillonite and the like), magnesium chloride, magnesium oxide, calcium sulfate, activated alumina, alumina, bauxite, anhydrous calcium sulfate, water-absorbent clay, and mixtures of these components and activated carbon. The desiccant is preferably one or more selected from the group consisting of silica gel, silica alumina gel (allophane), synthetic zeolite, and calcium chloride, and from the viewpoint of suppressing the interaction between the 5-ALA compound and moisture, the desiccant is still more preferably synthetic zeolite.

The shape of the desiccant may be, for example, a plate-shaped or bag-shaped sheet type, or may be cylindrical (tablet type). Also, the desiccant may be used as a film integrated with a resin.

The product can be produced, for example, by a method including a packaging process of producing a non-aqueous formulation, and then containing the non-aqueous formulation and a desiccant as necessary in the package. In the packaging process, device and means according to the form of the non-aqueous formulation and package can be appropriately used. The method for containing the non-aqueous formulation and the desiccant as necessary in the package may be, for example, a method in which the non-aqueous formulation and the desiccant as necessary are placed in the package, or may be a method in which the non-aqueous formulation is placed in the package (for example, jar) having a lid, and the desiccant (preferably, cylindrical (tablet type) desiccant) is attached to the lid. Alternatively, after the non-aqueous formulation is placed in a primary package (for example, PTP package), the primary package containing the non-aqueous formulation and the desiccant (for example, bag-shaped or sheet-shaped desiccant) may be further placed in a secondary package (for example, pillow type package).

Examples

Hereinafter, the present invention will be described more specifically with reference to formulation examples. However, the present invention is not limited to these formulation examples.

In Formulation Examples 1 to 40, the following components were used.
5-ALA hydrochloride: 5-Aminolevulinic acid hydrochloride

(Component A)

A-1: Isomalt hydrate (manufactured by BENEO-Palatinit GmbH)

A-2: D-mannitol (manufactured by Merck)
A-3: Anhydrous lactose (manufactured by DFE Pharma)
A-4: Lactose hydrate (manufactured by Freund Corporation)
A-5: Sorbitol (manufactured by Merck)
A-6: Trehalose (manufactured by Hayashibara Co., Ltd.)
A-7: Reduced maltose syrup (manufactured by Mitsubishi Corporation Life Sciences Limited)
a-1: Crystalline cellulose (manufactured by Asahi Kasei Corporation)
a-2: Corn starch (manufactured by JAPAN CORN STARCH CO., LTD.)
a-3: Low-substituted hydroxypropyl cellulose (manufactured by Shin-Etsu Chemical Co., Ltd.)

(Component B)

B-1: Sucrose fatty acid ester (manufactured by Mitsubishi Chemical Corporation, melting point: 58 to 64°C)
B-2: Glycerin fatty acid ester (manufactured by Riken Vitamin Co., Ltd., melting point: 68 to 73°C)
B-3: Stearic acid (manufactured by NOF Corporation, melting point: 56 to 72°C)
B-4: Hydrogenated rapeseed oil ("Lubriwax (registered trademark)" manufactured by Freund Corporation, 69 to 86°C)
b-1: Magnesium stearate (manufactured by TAIHEI CHEMICAL INDUSTRIAL CO., LTD.)
b-2: Calcium stearate (manufactured by TAIHEI CHEMICAL INDUSTRIAL CO., LTD.)
b-3: Sodium stearyl fumarate (manufactured by DuPont)

(Component C)

C-1: Hydroxypropyl cellulose (manufactured by Nippon Soda Co., Ltd.)
C-2: Hypromellose (manufactured by Shin-Etsu Chemical Co., Ltd.)

[Production of Non-Aqueous Formulations and Products]

<Formulation Examples 1 to 10, 14 to 23, 27 to 40>

Aminolevulinic acid hydrochloride (5-ALA hydrochloride) in the amounts shown in Tables 2 to 5 and component A in the types and amounts shown in Tables 2 to 5 were each put in a poly bag and mixed, then types and amounts of component B shown in Tables 2 to 5 were added and further mixed, and each mixture was molded in a circular tablet mold with a diameter of 5 mm using an automatic tableting tester (Auto-Tab-200TR type, manufactured by ICHIHASHI SEIKI KYOTO JAPAN) to prepare tablets (non-aqueous formulations) according to Formulation Examples 1 to 10, 14 to 23, and 27 to 40. The obtained tablets were accommodated in polyethylene bags (PE bags), and the tablets according to Formulation Examples 1 to 10 and Formulation Examples 27 to 33 were accommodated in only PE bags, and the PE bags containing the tablets according to Formulation Examples 14 to 23 and Formulation Examples 34 to 40 were each accommodated into a bag made of aluminum (aluminum bag) with desiccant to produce the products. Formulation Examples 27 to 40 are comparative examples.

<Formulation Examples 11, 24>

Amounts of 5-ALA hydrochloride, component A, and component C shown in Tables 2 and 3 were each charged into a vertical granulator (FM-VG-10, Powrex), 99.5% ethanol was each added thereto, and stirring granulation was performed. Thereafter, the granulated product was sized by a power mill (D-80 type, Dalton) and dried at 50°C by a blower constant temperature dryer (WFO-1020, TOKYO RIKAKIKAI CO., LTD.). After drying, the granules were each sized with a sieve with a mesh size of 850 μm to obtain granulated granules. The tablets (non-aqueous formulations) according to Formulation Examples 11 and 24 were prepared in the same manner as in Formulation Examples 1 to 10 and 14 to 23 except that the granulated granules prepared by the above methods were used, and the obtained tablets were accommodated in a PE bags. The tablets according to Formulation Example 11 were accommodated only the PE bags, and the PE bag containing the tablets for the tablets according to Formulation Example 24 and a desiccant were accommodated into an aluminum bag to produce the products.

<Formulation Examples 12, 13, 25, 26>

Amounts of 5-ALA hydrochloride, component A, and component C shown in Tables 2 and 3 were each charged into a vertical granulator (FM-VG-10, Powrex), 99.5% ethanol was each added thereto, and stirring granulation was performed. Thereafter, the granulated product was sized by a power mill (D-80 type, Dalton) and dried at 50°C by a blower constant temperature dryer (WFO-1020, TOKYO RIKAKIKAI CO., LTD.). After drying, the granules were each sized with a sieve with a mesh size of 850 μm to obtain granulated granules. The granulated granules prepared by the above method, and the types and amounts of component B shown in Tables 2 and 3 were each put in a poly bag and mixed, and then each mixture was molded in a circular tablet mold with a diameter of 5 mm using an automatic tableting tester (Auto-Tab-200TR type, manufactured by ICHIHASHI SEIKI KYOTO JAPAN) to prepare tablets (non-aqueous formulations) according to Formulation Examples 12, 13, 25, and 26. The obtained tablets were accommodated in PE bags, the tablets according to Formulation Examples 12 and 13 were accommodated in PE bags, and the PE bags containing the tablets for the tablets according to Formulation Examples 25 and 26 were each accommodated in an aluminum bag with a desiccant to produce the products.

[Temporal Stability Test]

The non-aqueous formulations according to each Formulation Example were stored in the form of products in an environment of 40°C and 75%RH for 2 months, and after the storage the content of a compound of the following formula (II) (also referred to as compound (II).), which is a representative analog of 5-ALA, was measured by liquid chromatography. It is well known the degradation pathway of 5-ALA (see Non-Patent Document 1) and that compound (II) are produced from 5-ALA and increased as impurities (see Patent Document 5).

[Chemical Formula 3]

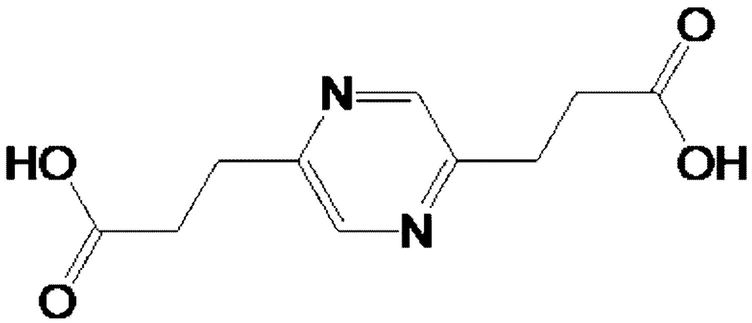

(II)

The conditions of liquid chromatography are as follows.
Detector: Ultraviolet absorption photometer (measurement wavelength: 260 nm)
Column: A stainless steel tube with an inner diameter of 3.0 mm and a length of 15.0 mm was packed with 3 μm of a reverse phase for liquid chromatography and strong cation exchange silica gel.
Column temperature: Constant temperature around 40°C
Mobile phase A: 0.3% Formic acid aqueous solution
Mobile phase B: 0.2 M Ammonium formate solution (water/acetonitrile (volume ratio) = 700/300)
Feeding of mobile phase: The concentration gradient was controlled by changing the mixing ratio of the mobile phase A and the mobile phase B as shown in Table 1 below.

[Table 1]

| Time after the injection (min) | Mobile phase A (volume %) | Mobile phase B (volume %) |
|---|---|---|
| 0~10 | 90→25 | 10→75 |
| 10~25 | 25 | 75 |
| 25~25.01 | 25→90 | 75→10 |
| 25.01~45 | 90 | 10 |

Flow rate: 0.4 mL/min
Injection amount: 20 μL

Sample cooler temperature: 5°C

The peak area of the compound of the formula (I) (ATI) and the peak area of the compound of the formula (II) (ATII) were determined for 25 minutes after the sample solution injection, and the amount of the compound of the formula (II) was calculated by the following formula. The results are shown in Tables 2 to 5.

$$\text{Content (\%) of compound (II)} = (\text{ATII}/\text{ATI}) \times 100/165$$

In the above formula, 165 is a sensitivity coefficient.

[Table 2]

| | | Formulation Example 1 | Formulation Example 2 | Formulation Example 3 | Formulation Example 4 | Formulation Example 5 | Formulation Example 6 | Formulation Example 7 | Formulation Example 8 | Formulation Example 9 | Formulation Example 10 | Formulation Example 11 | Formulation Example 12 | Formulation Example 13 |
|---|---|---|---|---|---|---|---|---|---|---|---|---|---|---|
| **Quantity** (mass %) | 5-ALA hydro-chloride | 50 | 50 | 50 | 50 | 50 | 50 | 40 | 20 | 10 | 5 | 50 | 50 | 50 |
| | A-1 | 47 | - | - | - | - | - | - | - | - | - | - | - | - |
| | A-2 | - | 47 | - | - | - | - | 57 | 77 | 87 | 92 | 47 | - | - |
| | A-3 | - | - | 47 | - | - | - | - | - | - | - | - | - | - |
| | A-4 | - | - | - | 47 | - | - | - | - | - | - | - | - | - |
| | A-5 | - | - | - | - | 47 | - | - | - | - | - | - | - | - |
| | A-6 | - | - | - | - | - | 47 | - | - | - | - | - | - | - |
| | A-7 | - | - | - | - | - | - | - | - | - | - | - | 45.6 | 45.6 |
| | B-1 | 3 | 3 | 3 | - | - | - | 3 | 3 | 3 | 3 | 3 | 3 | 3 |
| | B-2 | - | - | - | 3 | - | - | - | - | - | - | - | - | - |
| | B-3 | - | - | - | - | 3 | - | - | - | - | - | - | - | - |
| | B-4 | - | - | - | - | - | 3 | - | - | - | - | - | - | - |
| | C-1 | - | - | - | - | - | - | - | - | - | - | - | 1.4 | - |
| | C-2 | - | - | - | - | - | - | - | - | - | - | - | - | 1.4 |
| Desiccant | | None | None | None | None | None | None | None | None | None | None | None | None | None |
| Content (%) of compound (II) | | 0.00 | 0.00 | 0.01 | 0.00 | 0.00 | 0.00 | 0.01 | 0.01 | 0.00 | 0.00 | 0.00 | 0.00 | 0.01 |

[Table 3]

| | | Formulation Example 14 | Formulation Example 15 | Formulation Example 16 | Formulation Example 17 | Formulation Example 18 | Formulation Example 19 | Formulation Example 20 | Formulation Example 21 | Formulation Example 22 | Formulation Example 23 | Formulation Example 24 | Formulation Example 25 | Formulation Example 26 |
|---|---|---|---|---|---|---|---|---|---|---|---|---|---|---|
| Quantity (mass %) | 5-ALA hydrochloride | 50 | 50 | 50 | 50 | 50 | 50 | 40 | 20 | 10 | 5 | 50 | 50 | 50 |
| | A-1 | 47 | - | - | - | - | - | - | - | - | - | - | - | - |
| | A-2 | - | 47 | - | - | - | - | 57 | 77 | 87 | 92 | 47 | - | - |
| | A-3 | - | - | 47 | - | - | - | - | - | - | - | - | - | - |
| | A-4 | - | - | - | 47 | - | - | - | - | - | - | - | - | - |
| | A-5 | - | - | - | - | 47 | - | - | - | - | - | - | - | - |
| | A-6 | - | - | - | - | - | 47 | - | - | - | - | - | - | - |
| | A-7 | - | - | - | - | - | - | - | - | - | - | - | 45.6 | 45.6 |
| | B-1 | 3 | 3 | 3 | - | - | - | 3 | 3 | 3 | 3 | 3 | 3 | 3 |
| | B-2 | - | - | - | 3 | - | - | - | - | - | - | - | - | - |
| | B-3 | - | - | - | - | 3 | - | - | - | - | - | - | - | - |
| | B-4 | - | - | - | - | - | 3 | - | - | - | - | - | - | - |
| | C-1 | - | - | - | - | - | - | - | - | - | - | - | 1.4 | - |
| | C-2 | - | - | - | - | - | - | - | - | - | - | - | - | 1.4 |
| Desiccant | | Exist | Exist | Exist | Exist | Exist | Exist | Exist | Exist | Exist | Exist | Exist | Exist | Exist |
| Content (%) of compound (II) | | 0.00 | 0.00 | 0.01 | 0.00 | 0.00 | 0.00 | 0.01 | 0.01 | 0.00 | 0.00 | 0.00 | 0.00 | 0.01 |

[Table 4]

| | | Formulation Example 27 | Formulation Example 28 | Formulation Example 29 | Formulation Example 30 | Formulation Example 31 | Formulation Example 32 | Formulation Example 33 |
|---|---|---|---|---|---|---|---|---|
| Quantity (mass %) | 5-ALA hydrochloride | 50 | 50 | 50 | 50 | 50 | 50 | 50 |
| | A-2 | - | - | - | 49 | 49 | 48 | - |
| | a-1 | 47 | - | - | - | - | - | 36 |
| | a-2 | - | 47 | - | - | - | - | 10 |
| | a-3 | - | - | 47 | - | - | - | 3 |
| | B-1 | 3 | 3 | 3 | - | - | - | - |
| | b-1 | - | - | - | 1 | - | - | 1 |
| | b-2 | - | - | - | - | 1 | - | - |
| | b-3 | - | - | - | - | - | 2 | - |
| Desiccant | | None | None | None | None | None | None | None |
| Content (%) of compound (II) | | 0.02 | 0.03 | 0.05 | 0.10 | 0.08 | 0.13 | 0.22 |

[Table 5]

| | | Formulation Example 34 | Formulation Example 35 | Formulation Example 36 | Formulation Example 37 | Formulation Example 38 | Formulation Example 39 | Formulation Example 40 |
|---|---|---|---|---|---|---|---|---|
| Quantity (mass %) | 5-ALA hydrochloride | 50 | 50 | 50 | 50 | 50 | 50 | 50 |
| | A-2 | - | - | - | 49 | 49 | 48 | - |
| | a-1 | 47 | - | - | - | - | - | 36 |
| | a-2 | - | 47 | - | - | - | - | 10 |
| | a-3 | - | - | 47 | - | - | - | 3 |
| | B-1 | 3 | 3 | 3 | - | - | - | - |
| | b-1 | - | - | - | 1 | - | - | 1 |
| | b-2 | - | - | - | - | 1 | - | - |
| | b-3 | - | - | - | - | - | 2 | - |
| Desiccant | | Exist | Exist | Exist | Exist | Exist | Exist | Exist |
| Content (%) of compound (II) | | 0.02 | 0.03 | 0.06 | 0.04 | 0.03 | 0.04 | 0.09 |

## Claims

1. A non-aqueous formulation comprising:

   a compound of the following formula (I) or a salt thereof: ]

   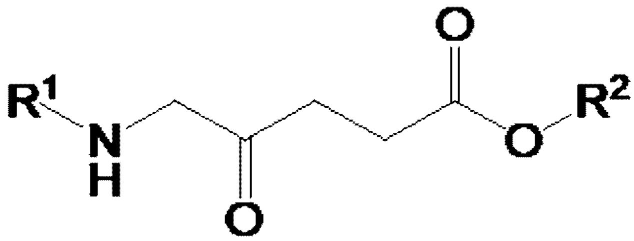

   (I)

   wherein $R^1$ is a hydrogen atom or an acyl group, and $R^2$ is a hydrogen atom, a linear or branched alkyl group, a cycloalkyl group, an aryl group, or an aralkyl group;
   a sugar or sugar alcohol; and
   at least one component having a melting point of 30 to 100°C selected from the group consisting of fatty acids, fatty acid esters, aliphatic hydrocarbons, and aliphatic alcohols .

2. The non-aqueous formulation according to claim 1, wherein the sugar or sugar alcohol is one or more selected from the group consisting of D-mannitol, erythritol, xylitol, sorbitol, lactose, trehalose, isomalt hydrate, and maltitol.

3. The non-aqueous formulation according to claim 1, wherein the fatty acid ester is a fatty acid ester of a polyhydric alcohol.

4. The non-aqueous formulation according to claim 1, wherein the fatty acid ester is one or more selected from the group consisting of sucrose fatty acid esters and glycerin fatty acid esters.

5. The non-aqueous formulation according to claim 1, wherein the fatty acid is a saturated fatty acid having 10 to 24 carbon atoms.

6. The non-aqueous formulation according to claim 1, wherein the content of the compound of the formula (I) or the salt thereof is 5 to 80 mass% based on the total amount of the non-aqueous formulation.

7. A product comprising the non-aqueous formulation of any one of claims 1 to 6 and a package that accommodates the non-aqueous formulation in a dry state.

8. The product according to claim 7, wherein the non-aqueous formulation has the form of tablets, capsules, powder, granules, or fine granules.

9. The product according to claim 7, wherein the non-aqueous formulation is brought into a dry state by one or more desiccants selected from the group consisting of silica gel, silica alumina gel, natural zeolite, synthetic zeolite, calcium chloride, quicklime, bentonite clay, magnesium chloride, magnesium oxide, calcium sulfate, activated alumina, alumina, bauxite, anhydrous calcium sulfate and water-absorbing clay, and mixtures of these components and activated carbon.

**INTERNATIONAL SEARCH REPORT**

International application No. **PCT/JP2023/024334**

**A. CLASSIFICATION OF SUBJECT MATTER**

***A61K 31/197***(2006.01)i; ***A61K 9/14***(2006.01)i; ***A61K 9/16***(2006.01)i; ***A61K 9/20***(2006.01)i; ***A61K 9/48***(2006.01)i; ***A61K 31/22***(2006.01)i; ***A61K 47/02***(2006.01)i; ***A61K 47/04***(2006.01)i; ***A61K 47/06***(2006.01)i; ***A61K 47/10***(2017.01)i; ***A61K 47/12***(2006.01)i; ***A61K 47/14***(2017.01)i; ***A61P 1/08***(2006.01)i; ***A61P 7/06***(2006.01)i; ***A61P 43/00***(2006.01)i

FI: A61K31/197; A61K9/14; A61K9/16; A61K9/20; A61K9/48; A61K31/22; A61K47/02; A61K47/04; A61K47/06; A61K47/10; A61K47/12; A61K47/14; A61P1/08; A61P7/06; A61P43/00

According to International Patent Classification (IPC) or to both national classification and IPC

**B. FIELDS SEARCHED**

Minimum documentation searched (classification system followed by classification symbols)

A61K31/197; A61K9/14; A61K9/16; A61K9/20; A61K9/48; A61K31/22; A61K47/02; A61K47/04; A61K47/06; A61K47/10; A61K47/12; A61K47/14; A61P1/08; A61P7/06; A61P43/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2015-54836 A (HAYASHIBARA, Masaaki) 23 March 2015 (2015-03-23)<br>claims, paragraphs [0017], [0043], example 4 | 1-4, 6-9 |
| A | claims, paragraphs [0017], [0043], example 4 | 5 |
| A | JP 5-112488 A (MUTENKA SHOKUHIN HANBAI KYODO KUMIAI) 07 May 1993 (1993-05-07)<br>example 1 | 1-9 |
| A | WO 2019/230939 A1 (KYUSHU UNIVERSITY, NATIONAL UNIVERSITY CORP.) 05 December 2019 (2019-12-05)<br>entire text | 1-9 |
| A | JP 2012-31163 A (SEIWA KK) 16 February 2012 (2012-02-16)<br>entire text | 1-9 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 September 2023** | **19 September 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/024334**

**C. DOCUMENTS CONSIDERED TO BE RELEVANT**

| Category* | Citation of document, with indication, where appropriate, of the relevant passages | Relevant to claim No. |
|---|---|---|
| A | JP 2010-240078 A (SBI ALAPROMO CO., LTD.) 28 October 2010 (2010-10-28) claims, paragraph [0022] | 1-9 |
| A | KR 10-2011-0138709 A (PAIK, Kyeong Seok) 28 December 2011 (2011-12-28) entire text | 1-9 |

Form PCT/ISA/210 (second sheet) (January 2015)

**INTERNATIONAL SEARCH REPORT**
**Information on patent family members**

International application No.
**PCT/JP2023/024334**

| Patent document cited in search report | Publication date (day/month/year) | Patent family member(s) | Publication date (day/month/year) |
|---|---|---|---|
| JP 2015-54836 A | 23 March 2015 | (Family: none) | |
| JP 5-112488 A | 07 May 1993 | (Family: none) | |
| WO 2019/230939 A1 | 05 December 2019 | US 2021/0205211 A1<br>whole document<br>EP 3808377 A1<br>CN 112203643 A<br>KR 10-2021-0028609 A | |
| JP 2012-31163 A | 16 February 2012 | (Family: none) | |
| JP 2010-240078 A | 28 October 2010 | (Family: none) | |
| KR 10-2011-0138709 A | 28 December 2011 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013054756 A
- WO 2013084816 A
- WO 2013054770 A
- WO 2018043240 A
- JP 2014189492 A


### Non-patent literature cited in the description


- **BUNKE, A. et al.** Degradation Mechanism and Stability of 5-Aminolevulinic Acid. *JOURNAL OF PHARMACEUTICAL SCIENCES*, October 2000, vol. 89 (10), 1335-1341